# EUROPEAN PATENT APPLICATION

(11) **EP 2 123 308 A1**
(43) Date of publication of application: **25.11.2009**
(21) Application number: 08155633.4
(22) Date of filing: 05.05.2008
(51) Int. Cl.: A61L 2/18, A61L 2/20

(54) **Sterilization by treatment with reductant and oxidant.**

(71) Applicant: B-K Medical ApS, 2730 Herlev (DK)
(72) Inventor: Sørensen, Jan, 3460 Birkerød (DK); Dueholm Uckevicius, Belinda, 2670 Greve (DK)
(74) Representative: Nilausen, Kim

(57) **Abstract**

The present invention relates to the field of sterilization of items that are sensitive to e.g. temperature, pH, positive or negative pressure, radiation or oxidation. More particularly, the invention concerns a method, the use of this method and an apparatus for sterilization or disinfection, comprising the steps of contacting one or more item or part of an item with a water-based fluid comprising at least one reductant, followed by the step of contacting with a gas having oxidative properties in a substantially water-free environment. According to the invention, items can be sterilized that are otherwise impaired by conventional sterilization procedures, such as laboratory items, medical items, dental items, military items, biological items, and food processing-related items.

## Description

### Technical field of the invention

The present invention relates to the field of sterilization of an item. More particularly, the invention concerns a method, the use of this method, and an apparatus for sterilization.

### Background of the invention

The healthcare segment concerning development of new and more dedicated equipment and tools is growing. Often, these become more and more complex, such as by the use of electronics inside the equipment in order to provide improved tools for examination and treatment. However, a large number of these items is also more fragile, and they often exhibit limited tolerances to elevated temperatures due to a complex material composition and assembly. When dealing with patients, materials and items used during treatment, for example surgery and or examination, often come into direct contact with the patient. When reusing these materials or equipments, they have to be sterilized or disinfected in order to avoid patient to patient contamination.

The most used method for sterilisation is autoclaves, where high temperature, often in combination with steam, is used for sterilization. Often, the temperatures in an autoclave exceed 121°C or higher, resulting in damage of temperature sensitive equipment.

Therefore, there is a need for alternative sterilization methods for temperature sensitive items. This need is not restricted to the medical sector, and sterility is a necessity in other environments and applications, such as microbiology, military, industrial fermentations, and for food processing areas including slaughter houses.

Other methods are using plasma and/or gas as sterilization agent in combination with vacuum. However, the underpressure or negative pressure generated in these processes will damage equipment with closed cavities, such as ultrasound transducers and endoscopes.

Often, an item or object is considered sterile, when it is free of all living microorganisms, and this state of sterility is the result of a sterilizing procedure. This may be a chemical and/or physical process destroying and/or eliminating all living organisms, which also includes resistant bacterial spores. As it is practically impossible to prove this desired condition, the result of a sterilizing process is defined as a probability of less than one in one million that a microorganism has survived on an item. This is also referred to as "sterility assurance level" and is used by the medical device industry to characterize sterilized medical devices. In practical terms and in view of the current invention, sterilization is defined as a 6 log reduction in microbial load.

Usually, sterilization of objects/items with solid or semisolid surfaces is achieved by surface sterilization. In contrast, liquids and/or gel-like compositions require methods of sterilization with penetrating potential.

Disinfection on the other hand, not to be confused with sterilization, is defined as the process of destroying or inhibiting growth of microorganisms. There are three stages of disinfection, low-level, intermediate-level and high-level disinfection. Low-level disinfection kills vegetative, i.e. growing bacteria, fungi and susceptible viruses, while intermediate-level disinfection kills most bacteria, fungi and viruses, but not bacterial spores. High-level disinfection kills all bacteria, fungi, viruses and may kill bacterial spores, especially when for example prolonged contact times are chosen.

Bacterial spores, or "spores" are considered the most resistant of all living organisms because of their ability to withstand a variety of chemical and/or physical processes, which otherwise are capable of effectively destroying all other living organisms. Fungi have also the ability to produce spores, but in contrast to bacterial spores, fungal spores are less resistant.

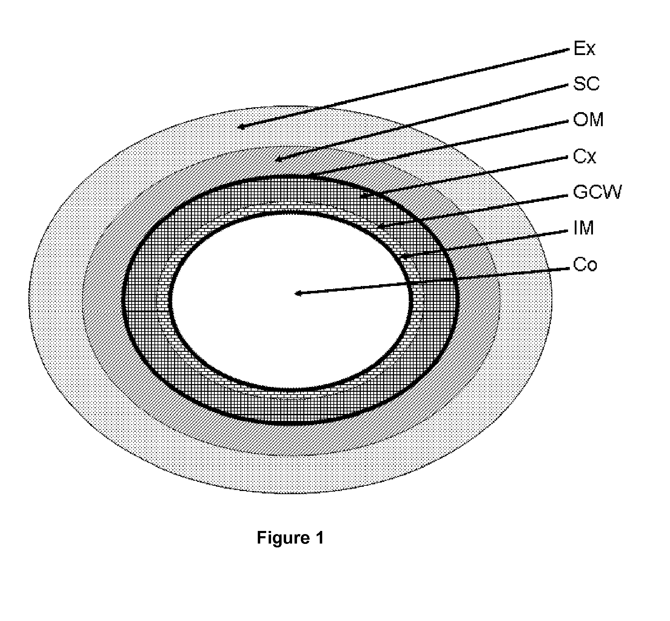

In the following, the current knowledge about the mechanisms involved with respect to resistance of bacterial spores towards sterilization is discussed in more details. Spores are differentiated cells formed within a vegetative bacterial cell in response to unfavourable environmental conditions. It has been reported that spores are several orders of magnitude more resistant to lethal treatments and/or chemical agents than its parent cell. A cross section of a bacterial spore is presented in Fig. 1. The spore is often surrounded by a covering known as the exosporium (Ex), which overlies the spore coat (SC). The spore coat is a complex, multi-layered structure consisting of more than 50 proteins. The spore coat is the major resistance barrier for a large number of chemicals, such as most oxidizing agents including chlorine dioxide, hypochlorite, ozone and peroxynitrite, but not against heat or radiation. The layered outer coats of a bacterial spore are rather inert and play a predominant role in protecting the spore against exogenous agents. It is known that disulfide bridges are a feature of cellular walls and other protein-containing features of bacterial cells. As much as 80% of the total protein of the spore is made up of keratin-like protein. The stability of keratin structures is due to frequent valence cross links (disulfide bonds) and secondary valence cross links (hydrogen bonds) between neighboring polypeptide chains. Keratin-like proteins are resistant to proteolytic enzymes and hydrolysis, but typically insoluble in aqueous salt solutions or dilute acid or base solutions. The cortex (Cx) which consists of peptidoglycan lies beneath the spore coat separated by the outer membrane (OM). The inner membrane (IM) is located between the core wall and the cortex, and surrounds the core (Co) of the endospore. The inner membrane exhibits an extremely low permeability to small hydrophobic and hydrophilic molecules. The core contains normal cell components, such as DNA and ribosomes, but it is considered metabolically inactive.

The mechanisms involved in spore resistance towards sterilization by heat, drying, freezing, toxic chemicals or radiation are not completely understood. The current scientific knowledge is summarized in the recent review article "Spores of Bacillus subtilis: their resistance to and killing by radiation, heat and chemicals") by Setlow (2006; Journal ofApplied Microbiology, 101, 514-525.) Setlow summarizes the different factors that contribute to spore resistance towards physical and chemical sterilization. These are (i) significantly reduced water content, (ii) the level and type of spore core mineral ions, (iii) the intrinsic stability of total spore proteins, (iv) saturation and protection of DNA with acid-soluble spore proteins (SASP), (v) DNA repair agents, (vi) alteration in spore DNA photochemistry, (vii) spore coat proteins and, (viii) relative impermeability of the spore inner membrane.

*Bacillus stearothermophilus* is a thermophilic species which can grow at temperatures at 65°C or above. Spores of *Bacillus stearothermophilus* are highly temperature resistant and they are used for example as sterility indicators for steam sterilization. Commercial indicators from FLUKA consist of 1 million spores impregnated on paper strips. These indicators are specified by US military specification MIL-S-36586 and are GMP (Good Manufacturing Practice) requirements of the US FDA (Food and Drug Administration). While *Bacillus stearothermophilus* is mainly used for testing sterilization at high temperatures, *Bacillus atrophaeus* is used as indicator for sterilization at low temperature. The Commercial indicators can be obtained from Raven Biological Laboratories, INC and consist of 1 million spores on stainless steel discs. This microorganism is also part of an US FDA-approved method (FDA Guidance on Premarket notification 510K submissions for Sterilizers intended for use in the health care facilities).

Sterilization procedures can be divided in two major groups, namely physical and chemical processes.

Common physical sterilization procedures are based on heat, either dry heat or moist heat, based on saturated steam, often in combination with pressure. This is for example the major principles in autoclaves, a common instrument used for sterilization.

Heat destroys microorganisms, and their death is caused by denaturation of proteins and enzymes in the cells. This process is accelerated by addition of moisture. Although most vegetative forms of microorganisms are killed in a few minutes at 65°C, certain bacterial spores can withstand temperatures of 115°C for more than 3 hours. It is believed that no living organism can survive direct exposure to saturated steam at 121°C for more than 15 minutes.

In the absence of moisture, dry heat in the form of hot air requires higher temperatures. Death of microorganisms is again the result of denaturation of proteins and enzymes, combined with oxidation processes. Minimum time requirements for sterilization depend on the temperature used, ranging e.g. from one hour at 171 °C or six hours at 121°C.

Radiation may also be used for sterilization and/or disinfection. UV light, form of non-ionizing radiation, is used for sterilization at room temperature, but it is limited to surfaces and some transparent objects. UV is mainly used for sterilizing the interiors of biological safety cabinets between uses. UV is ineffective for sterilization in shaded areas, e.g. cavities or areas under dirt. UV damages many plastics.

Microwave can also be used for sterilization, where the non-ionizing radiation produces energy rich hyperthermic conditions that disrupt life by acting on water molecules, thereby disrupting e.g. cell membranes. Short sterilization cycles of few minutes at only slightly elevated object temperatures can be achieved. Not all objects are suited for sterilization by microwave.

Energy-rich, ionizing radiation in form of β-particles, X-rays or γ-rays is routinely used mainly for batch sterilization, where the ionic energy of the radiation is converted to thermal and chemical energy. Sterilization cycles are long, often overnight. Major advantage is the ability of the ionizing radiation to penetrate through larger objects. Furthermore, dosimetry can be used for immediate assessment of the efficiency of the sterilization process, instead of depending on tedious microbiological tests.

Chemical sterilization procedures are usually applied when the items to be sterilized are heat or moisture sensitive, i.e. when they cannot be sterilized in a dry or steam autoclave. Common chemical agents that are used for killing microorganisms including bacterial spores are ethylene oxide gas, formaldehyde gas, glutaraldehyde activated solution or gas, hydrogen peroxide plasma/vapor, peracetic solution, bleach and ozone gas or in aqueous solution. The major disadvantages of chemical sterilization are (i) the toxicity and (ii) flammability/explosive danger of the chemical compounds used, (iii) the need for aeration of sterilized items after sterilizitation (iv) their often aggressive and corrosive properties towards e.g. plastics and metals, and finally, (v) the long sterilization times needed.

In view of issues related to the use of chemical sterilization and the time requirements of physical sterilization procedures, there is a need for a rapid (< 1 hour, 30 min or less than 30 min), flexible, preferably non-toxic, reliable and simple procedure for sterilizing items that cannot be sterilized by steam, i.e. items that are impaired by temperature or other forms of chemical and/or physical sterilization.

PCT/DK/2007/000486, a patent application submitted by the same applicant, concerns a method and apparatus for two-step sterilization, comprising the steps of contacting an item or part of an item with a water-based fluid containing at least one enzyme, and a substantially water-free environment with a gas having oxidative properties, such as ozone.

Surprisingly and unexpectedly, the applicant has discovered a process for sterilization and/or disinfection suitable for killing bacterial spores, said process comprising the steps of (i) treatment with a water-base fluid comprising a reductant, followed by a (ii) treatment with a fluid comprising an oxidant.

### Summary of the invention

The invention solves the problem of sterilizing and/or disinfecting items which are prone to be impaired or damaged by for example one or more of temperature, pH, positive or negative pressure, radiation, oxidation, detergent and/or enzymatic activity.

A first aspect of the invention concerns a method for sterilization, comprising the steps of contacting an item or part of an item with a water-based fluid containing a reductant, followed by contacting said item or part of an item with a gas having oxidative properties in a substantially water-free environment, thereby providing sterilization and/or disinfection of said item or part of said item.

A second aspect of the invention concerns the use of said method, such as treatment of one or more items, or one or more parts of an item selected from the group consisting of laboratory item, medical item, dental item, military item, biological item, food processing related item, satellite and space rocket.

A third aspect of the invention relates to an apparatus comprising the necessary means for performing, using or applying said method.

### Detailed description of the invention

The present invention, which completely or partially satisfies the abovementioned objectives, relates to a method comprising two distinct steps/treatments for sterilization and/or disinfection. Without being bound or limited by any theory, it is believed that the first step (a) renders a layer or fraction or subfraction of a layer or coat of the microbe or bacterial spore susceptible for the sterilizing/disinfecting action of the second treatment (b).

The inventors surprisingly and unexpectedly discovered that sterilization and/or disinfection was achieved by treatments with an aqueous fluid under reducing conditions, such as water comprising a reductant, followed by treatment under oxidizing conditions, such as a treatment with a gaseous fluid comprising an oxidant, such as air comprising ozone. This was feasible at temperatures well below the boiling point of water. Furthermore, sterilization can be achieved in a surprisingly short time. Finally, this method proves suitable for sterilization of items or part of items that are not suitable for conventional sterilization/disinfection procedures.

Thus, in a first aspect, the invention relates to a method according to claim 1, providing a method for sterilization or disinfection, comprising the steps of contacting one or more item or part of said item with (a) a water-based fluid comprising at least one reductant, and (b) a substantially water-free environment with a gas having oxidative properties, wherein step (a) precedes step (b).

In one embodiment of the invention, the method comprises a further step (a*) of contacting the one or more item or part of said item to be sterilized with a water-based fluid comprising at least one enzyme, wherein said step (a*) precedes step (b). According to another embodiment of the invention, step (a*) precedes step (a). In a further embodiment, step (a) precedes step (a*).

According to one embodiment of the invention, the method suitable for sterilization and/or disinfection comprising the steps of contacting one or more item or part of an item with a water-based fluid containing a reductant, and in another distinct step which is performed after the previous step, contacting the one or more item or part of an item with an oxidant, such as a gas with oxidative properties in a substantially water-free environment. Such steps or treatments may be repeated for maximizing efficiency.

A water-base fluid according to the invention comprises water as the main component, such as at least 80%, 90%, 95%, 99%, or 99.5% (volume/volume) water. The water-based fluid comprise one or more of component, chemical composition, enzyme, additive and the like in any form, such as e.g. as solution, suspension, dispersion, and/or emulsion, or any combination of solution, suspension, dispersion, and/or emulsion of one or more of any component, composition, enzyme, additive and the like. The water-based fluid can be 100% water, such as distilled water, or clean water, such as filtered and/or de-ionized water. According to one embodiment of the invention, the water based fluid is based on processed water.

In one embodiment of the invention, step (a) and/or (a*) comprise(s) an ultrasonic treatment. In yet another embodiment, treatment with a water-based fluid comprises one or more of ultrasonic treatment, sonication, mixing, vortexing, moving, agitating and/or pumping an aqueous fluid. In another embodiment of the invention, the ultrasonic treatment/sonication, and/or mixing, vortexing, moving and/or pumping liquid solubilises, dissolves, and/or distributes compounds or particles within said water-based fluid. These compounds and/or particles (including spores, microorganisms, biological material and/or dirt) can be removed, or partially removed from the surface of one or more items to be sterilized or disinfected. In one embodiment of the invention, said ultrasonic treatment is facilitating this process.

According to one embodiment of the invention, one or more rinsing step(s) is included before step (a), step (a*), and/or step (b). Such a rinsing and/or washing step can comprise rinsing with processed water and/or processed gas. Commonly, but not necessarily, one or more rinsing steps can be provided between different steps of treatment, such as between treatment with a water based fluid comprising a reductant, and treatment with a gas with oxidative properties. Such one or more rinsing steps may consist of rinsing, flushing or treatment with processed water and/or processed gas, and/or other methods known in the art. The purpose of such a rinsing step can be removal of one or more undesired compounds, including dirt, biological material, contamination, chemical composition, buffer, buffering agent, acid, base, salt, OH⁻, H⁺, reductant, oxidant, water, enzyme, enzyme inhibitor, detergent, and the like, and any combination thereof. In one embodiment, the biological material is one or more of (biological) tissue, biomass, living or dead biological matter, biomaterial, bio-compatible material, bio-applicable material; biomolecule (i.e. a chemical compound or composition that naturally occurs a living organism), biotic material, processed biotic material, cellular component, material/substances of which a cell is composed, organic material/matter, viable material (i.e. material capable of living, developing, and/or germinating under appropriate condition), and/or body fluid.

Processed water according to the present invention can be e.g. demineralised water, tap water or sterile water. Processed water does not need to be sterile, if the microorganisms present therein are killed by the subsequent ozone treatment. In one embodiment of the invention, the processed water is sterile-filtered, such as passing said water through a filter with a defined maximal pore size. A filter with pore size 0.2 µm can effectively remove bacteria. If viruses must also be removed, a much smaller pore size may be needed, such as around 20 nm or less. In another embodiment of the invention, the processed water is sterilized by heating, such as autoclaving, or by a step of evaporation followed by condensation. Alternatively, the processed water can be sterilized by a combination of any treatments known in the art, or combination of treatments known in the art, or any of the aforementioned steps or processes. According to the invention, processed water can comprise one or more additives. According to a further embodiment of the invention, the aqueous fluids in step (a) and (a*) are based on processed water, where e.g. one or more of reductant(s), enzyme(s), additive(s), and the like, and any combination thereof are added.

According to one embodiment of the invention, the method comprises one or more rinsing step(s) which comprises contacting said one or more item or part of said item with processed water and/or processed gas.

By flushing or rinsing or washing with an appropriate gas, a substantially water-free environment is obtained, especially when the processed gas contains a low level of humidity. A substantially water-free environment can be defined as an environment with a low level of humidity, such as the lumen and/or inner surfaces of a chamber. This can also refer to a reaction chamber or container, its lumen and/or its content, which does not contain visible traces of humidity (such as droplets or pools of water). Alternatively, a substantially water-free environment refers to the content of a container or reaction chamber after removal of water-based fluid. Low level of humidity means less than 60%, 50%, 40%, 33%, 25%, 20%, 15%, 10%, 5%, 2%, 1%, 0.1% or 0.01% relative humidity. In one embodiment of the invention, low level of humidity refers to ambient humidity, such as around 40-60% relative humidity. Such a processed gas can be sterile, sterile-filtered, and/or sterilized (e.g. by UV). Processed gas can for example be dry or dried air, ambient air or nitrogen, and this gas could also comprise ozone. In an embodiment of the invention, the processed gas is ambient air with a low level of humidity, i.e. a lower level of humidity than ambient air. In a further embodiment, the processed gas does not contain any spores or other forms of life that are not killed by the succeeding ozone treatment. A processed gas comprising or consisting of dry or dried air can provide a substantially water-free environment according to the invention. According to one embodiment of the invention, the processed gas is sterile-filtered, such as passing said gas through a filter with a defined maximal pore size. A filter with pore size 0.2 µm is believed to effectively remove bacteria. If viruses must also be removed, it can be appropriate to use a smaller pore size, such as around 20 nm or less. Alternatively, the processed gas can be sterilized by a combination of any treatments known in the art, or combination of treatments known in the art, or any of the aforementioned steps or processes disclosed in this specification.

In the context of the present invention, the term "reductant" can be used interchangeably with the term "reducing agent" and/ or "reducer", and is meant to comprise a chemical composition with a negative redox potential compared to a standard hydrogen electrode, also called normal hydrogen electrode. In a redox reaction, the reductant is oxidized, while the partner in said redox reaction is reduced.

In one embodiment of the invention, a reductant is a chemical composition capable of reducing a disulfide bridge, such as a disulfide bridge between two cysteins. A disulfide bridge can be an intra-molecular disulfide bridge, i.e. a disulfide bridge within a chemical composition, such as a protein molecule. Alternatively, a disulfide bridge can be formed between two different molecules, e.g. proteins, also called inter-molecular disulfide bridge. Said two different molecules can be of identical, similar or different. A molecule can have one or more, inter- and/or intramolecular disulfide bridges. Commonly, disulfide bridges can be formed between two sulphur containing amino acids. Cystein is an amino acid comprising a thiol side chain. When incorporated in a protein, the cystein is capable of forming a disulfide bridge to a second cystein. Because of the high reactivity of this thiol, cysteine is believed to be an important structural and functional component of many proteins and enzymes. A cystein-dimer, linked via a disulfide bridge is also called cystine.

In one embodiment of the invention, the reductant capable of reducing a disulfide bridge comprises one or more chemical composition known in the art for being capable of breaking and/or modifying an intra- or inter-molecular disulfide bridge, e.g. between to cysteins. In another embodiment, the reductant is selected from the group consisting of on or more of β-mercaptoethanol, dithiothreitol (DTT), ascorbic acid, quinone, polyphenol with up to hundreds of polymeric subunits including but are not limited to phenol-rich polymers of flavonoids, gallic acid, ellagic acids and their respective carbohydrate esters, salts and derivatives, proanthocyanidin, including their free acid forms, derivative DTT, derivative of β-mercaptoethanol, and any combination thereof. In another embodiment of the invention, the reductant is one or more of dithiothreitol, a derivate of dithiothreitol, β-mercaptoethanol, a derivate of β-mercaptoethanol, and any combination thereof.

In one embodiment of the invention, the chemical composition known to be capable of modifying a thiol group is present in a concentration (weight/volume) of about 0.001 % to 5%, or 0.001 % to 1 %, or in a concentration of about 0.005% to 0.5%. In yet another embodiment of the invention, the aqueous fluid in step (a) comprises a reductant in a concentration (weight/volume) of less than 0.001 %; 0.001 % to 10%; 0.001 % to 0.005%; 0.005% to 0.01%; 0.01 % to 0.05%; 0.05% to 0.1%; 0.1 % to 0.5%; 0.5% to 1 %; 1 % to 2%; 2% to 5%; or 5 to 10%; or more than 10%. In a further embodiment, the total concentration of reductants in step (a) is less than 0.001 %; 0.001 % to 10%; 0.001 % to 0.005%; 0.005% to 0.01 %; 0.01 % to 0.05%; 0.05% to 0.1 %; 0.1 % to 0.5%; 0.5% to 1%; 1% to 2%; 2% to 5%; or 5 to 10%; or more than 10%. In yet a further another embodiment, a reductant is present in step (a) in a molar concentration of 1 mM or less; 1-2 mM; 2-5 mM; 5-10 mM; 10-50 mM; 50-100 mM; 0.1-0.5 M; 0.5 to 1 M; or more than 1 M. I about 0.005% to 0.5%. In yet another embodiment, the total molar concentration of reductants present in step (a) is 1 mM or less; 1-2 mM; 2-5 mM; 5-10 mM; 10-50 mM; 50-100 mM; 0.1-0.5 M; 0.5 to 1M; or more than 1 M.

In a further embodiment, upon reducing of the disulfide bridge by a reductant, the resulting thiol group is modified in order to prevent (re-)creation of a new disulfide bridge. Such a modification of a thiol group can comprise the use of one or more chemical composition(s) known in the art, which is/are known to be capable of modifying a thiol group, such as iodoacetamide and iodoacetate. In yet a further embodiment, the chemical composition known to be capable of modifying a thiol group is a derivate of iodoacetamide and/or iodoacetate.

In one embodiment of the invention, an alkaline pH is provided and/or maintained during treatment with an aqueous fluid comprising one or more reductant(s), and/or one or more enzyme(s), such as a pH greater than 7.1; 7.5; 8.0; 9.0; 10.0; 11.0; 12.0; 13.0 and/or 14.0. In another embodiment of the invention, an acid pH is provided and/or maintained during reductant and/or enzyme treatment, such as a pH less than 6.9; 6.0; 5.0; 4.0; 3.0; 2.0; and/or 1.0. In yet another embodiment of the invention, a near neutral pH is maintained and/or provided during reductant and/or enzyme treatment, such as a pH range of 6.0-8.0; 6.5-7.5, and/or 6.8-7.2.
According to one embodiment of the invention, the pH of a water-based fluid, including processed water, can be in the range of pH 2.0-12.0; 4.0-10.0, 6.0-8.0, or 6.8-7.2. Generally, it can be appropriate to maintain a constant pH during one step, and it can be appropriate to change the before or during another treatment step. Thus, the pH of the various fluids used during sterilization or disinfection may be similar, or very different. In one embodiment, for example a treatment step comprising an enzymatic treatment, the pH is in the area of the pH optimum of the enzyme, such as +/-1.0, 0.5, or 0.2 around the enzyme's pH optimum, where the pH optimum has been established in the same or similar buffer and/or reaction conditions.

In yet another embodiment, a constant pH, i.e. a pH +/- 0.05, or a near constant pH (+/- 0.2) is maintained during reductant and/or enzyme treatment. In yet a further embodiment, the pH is not maintained constant or near constant. A defined and/or desired pH can be achieved by providing titration with an acid or a base. Alternatively, an appropriate buffer, also called buffer solution, can be provided. Such a buffer commonly comprises one or more buffering agent, such as weak acid(s) and/or weak base(s).

According to one embodiment of the invention, step (a) is performed at a pH in the range of pH 6.0 to 14.0; 7.0 to 10; 7.0 to 8.0. According to another embodiment of the invention, step (a*) is performed at a pH in the range of pH 4 to 10; 6 to 8; or 6.8 to 7.2. According to a further embodiment, a rinsing step is performed at a pH of in the range of pH 6.0 to 14.0; 7.0 to 10; or 7.0 to 8.0; or pH 6.0 to 14.0; 7.0 to 10; or 7.0 to 8.0. According to yet another embodiment, step (a), (a*) and/or a rinsing/washing step is performed at a pH in the range of 1.0 to 2.0; 2.0 to 3.0; 3.0 to 4.0; 4.0 to 5.0; 5.0 to 6.0; 6.0 to 7.0; 7.0 to 8.0; 8.0 to 9.0; 9.0 to 10.0, 10.0 to 11.0; 11.0 to 12.0; 12.0 to 13.0;or 13.0 to 14.0. According to yet a further embodiment, step (a), (a*) and/or rinsing/washing step are performed at around the same pH or similar pH, or at a different pH.

Such buffering agents and/or buffers are known in the art, and they can comprise chemical compositions like TAPS (3-{[tris(hydroxymethyl)methyl] amino}propanesulfonic acid); Bicine (N,N-bis(2-hydroxyethyl)glycine), Tris (tris(hydroxymethyl)methylamine), Tricine (N-tris(hydroxymethyl) methylglycine), HEPES (4-2-hydroxyethyl-1-piperazineethanesulfonic acid), TES (2-{[tris(hydroxymethyl)methyl]amino} ethanesulfonic acid), MOPS (3-(N-morpholino)propanesulfonic acid), PIPES (piperazine-N,N'-bis(2-ethanesulfonic acid), cacodylate (dimethylarsinic acid), MES (2-(N-morpholino)ethanesulfonic acid), acetate, citrate, carbonate, phosphate, ammonium, a salt of an organic or inorganic acid, a salt of an organic or inorganic base, and the like. According to one embodiment of the invention, a buffer is provided in step (a), (a*) and/or during a washing/rinsing step, said buffer comprising one or more of the aforementioned buffers and/or buffering agents, and any combination thereof.

In the context of the present invention, the term "oxidant" can be used interchangeably with the term "oxidizing agent", and is meant to comprise a chemical composition with a positive redox potential compared to a standard hydrogen electrode. In a redox reaction, the oxidant is reduced, while the partner in the redox reaction is oxidized.

Generally, the term "redox" is meant to comprise an abreviation for reduction/oxidation. A "redox reaction" is meant to describe a chemical reaction comprising a change in oxidation state and/or oxidation number of one or more chemical compositions, such as molecules, atoms and/or ions involved. In the context of the present invention, a redox reaction can comprise simple redox process such as the oxidation of carbon to yield carbon dioxide, or the reduction of carbon by hydrogen to yield methane (CH₄), or the like. Alternatively, it can be one or more complex process(es), such as the oxidation of a sugar in a cell through a series of potentially complex electron transfer processes. The term "redox" comes from the two concepts of reduction and oxidation. It can be explained in simple terms,
where oxidation describes the loss of electrons by a chemical composition, such as molecule, atom and/or ion; while reduction describes the gain of electrons by a chemical composition, molecule, atom and/or ion. Alternatively, oxidation and reduction can also refer to a change in oxidation number, and the actual transfer of electrons may never occur. Thus, oxidation can be also defined as an increase in oxidation number, and reduction as a decrease in oxidation number. Commonly, a transfer of electrons will cause a change in oxidation number, but there are reactions which are classed as "redox" even though no electron transfer occurs, such as those involving covalent bonds.

According to one embodiment of the invention, the sterilization process comprises a third step, comprising the use of a water-based fluid containing one or more enzymes. According to one embodiment of the invention, addition of such a step comprising at least one enzyme improves disinfection or sterilization, such as increase in log reduction and/or reduction of time necessary for providing disinfection or sterilization.

According to one embodiment of the invention, the sterilization process can also comprise a step comprising where the water-based fluid comprises one or more reductant(s) and one or more enzymes. According to a further embodiment of the invention, one or more enzymes can be added at any time to e.g. the aqueous fluid comprising one or more reductant. If appropriate, suitable additives can be added in order to provide favourable reaction conditions for the one or more enzyme(s). This can also comprise providing a change in pH. According to yet another embodiment, one or more reductant(s) can be added to the aqueous fluid comprising one or more enzymes. If appropriate, suitable additives can be added in order to provide favourable or more favourable reaction conditions for the one or more reductant(s). This can also comprise providing a change in pH.

Such an enzyme can be selected from the group comprising microbial cell wall modifying or degrading enzymes, protein modifying or degrading enzymes and/or fat modifying or degrading enzymes. Without limitation, these enzymes can be selected from the group comprising cellulases, chitinases, amylases, proteases, lysozyme(s), phosphatases, kinases and/or lipases. In an embodiment of the invention, the enzyme or enzymes are capable of degrading the cell wall of bacterial spores. More precisely and non exclusively, the enzyme or enzymes are capable of degrading or impairing at least in part the exosporium, the spore coat, the outer membrane, the spore coat and/or the inner membrane. It is believed that these enzyme or enzymes are capable of impairing the spore and/or the protective layer(s) of the spores to such a degree, that the subsequent treatment with a gas containing ozone results in sterilization and/or disinfection according to the invention.

In a further embodiment of the invention, the enzymatic treatment comprises incubation with one or more enzymes selected from the group consisting of cellulose; chitinase; amylase; protease; lysozyme; phosphatase; kinase; lipase; sulfatase (a type of esterase enzyme which removes sulfate from a variety of substrates. Ex. Galactosamine-6 sulfatase, N-acetylglucos-amine-6-sulfatase), neuraminidase (a glycoside hydrolase enzyme (EC 3.2.1.18). It is frequently found as an antigenic glycoprotein and is best known as one of the enzymes found on the surface of the Influenza virus), aminopeptidase (a zinc-dependent enzyme produced by glands of the small intestine and assists in the enzymatic digestion of proteins therein), a digestive enzyme produced by a gland of a mammal, such as dipeptidase, maltase, sucrase, lactase, and enterokinase; achromopeptidase (a lysyl endopeptidase with a MW of ∼27kD. It is useful for lysis of Gram-positive bacteria that are resistant to lysozyme); lysostaphin (a Staphylococcus simulans metallo-endopeptidase. It can function as an antimicrobial against Staphylococcus aureus. Lysostaphin is a zinc endopeptidase with a molecular weight of approximately 25 kDa. Because lysostaphin cleaves the poly-glycine crosslinks in the peptidoglycan layer of the cell wall of Staphylococcus species it has been found useful for cell lysis); labiase from Streptomyces fulvissimus is an enzyme preparation useful for the lysis of many Gram-positive bacteria such as Lacto-bacillus, Aerococcus and Streptococcus. Labiase contains β-N-acetyl-D-glucosaminidase and lysozyme activity); mutanolysin provides gentle cell lysis for the isolation of easily degradable biomolecules and RNA from bacteria. It has been used in the formation of spheroplasts for isolation of DNA. Mutanolysin is a 23kD N-Acetyl Muramidase, like lysozyme, is a muralytic enzyme that cleaves the N-acetylmuramyl-β(1-4)-N-acetylglucosamine linkage of the bacterial cell wall polymer peptidoglycan-polysaccharide. Its carboxy terminal moieties are involved in the recognition and binding of unique cell wall polymers. Mutanolysin lyses Listeria and other gram positive bacteria such as Lactobacillus and Lactococcus); endochitinase A (an enzyme that breaks down glycosidic bonds in chitin. It is found in chitinivorous bacteria (bacteria that are able to digest chitin); chitobiosidase (another chitin degrading enzyme); N-Acetyl-beta-Glucos-aminidase; hyaluronidase (Chondroitin); chondroitinase (ABC, AC or C); cysteine proteases have a common catalytic mechanism that involves a nucleophilic cysteine thiol in a catalytic triad. The first step is deprotonation of a thiol in the enzyme's active site by an adjacent amino acid with a basic side chain, usually a histidine residue. The next step is nucleophilic attack by the deprotonated cysteine's anionic sulfur on the substrate carbonyl carbon. In this step, a fragment of the substrate is released with an amine terminus, the histidine residue in the protease is restored to its deprotonated form, and a thioester intermediate linking the new carboxy-terminus of the substrate to the cysteine thiol is formed. The thioester bond is subsequently hydrolyzed to generate a carboxylic acid moiety on the remaining substrate fragment, while regenerating the free enzyme. Ex. Papain, Cathepsins, Caspases, Calpains); caspases: As proteases, they are enzymes that cleave (cut) other proteins. They are called cysteine proteases, because they use a cysteine residue to cut those proteins, and called caspases because the cysteine residue cleaves their substrate proteins at the aspartic acid residue; Germination protease (GPR; an atypical aspartic acid protease located in spore coats); aspartyl protease (a protease which utilizes an aspartic acid residue for catalysis of their peptide substrates. They typically have two highly-conserved aspartates in the active site and are optimally active at acidic pH. Nearly all known aspartyl proteases are inhibited by pepstatin. Ex. HIV-1 protease - a major drug-target for treatment of HIV, chymosin (or "rennin", Renin (with one "n"), cathepsin D, pepsin, plasmepsin, aspartic protease precursor pepsinogen); keratinase and or keratanase. According to one embodiment of the invention, one ore more enzymes used during a step of enzymatic treatment, such as step (a*) can be selected from one or more of the aforementioned enzymes, including any combination.

In one embodiment of the invention, one or more enzymes used during a step of enzymatic treatment, such as step (a*) is selected from the group of disulfide-bridge altering enzyme, capable of cleaving, linking and/or re-aranging one or more disulfide bridges. Examples of such enzymes that can be suitable according to the invention comprise the following enzyme classes: glutathione-disulfide reductase (EC 1.8.1.7; also known as glutathione reductase, glutathione reductase (NADPH), glutathione S-reductase, GSH reductase, GSSG reductase, NADPH-glutathione reductase, NADPH-GSSG reductase, NADPH:oxidized-glutathione oxidoreductase); protein-disulfide reductase (EC 1.8.1.8, also known as disulfide reductase, insulin-glutathione transhydrogenase, NAD(P)H:protein-disulfide oxidoreductase); thioredoxin-disulfide reductase (EC 1.8.1.9, also known as NADP-thioredoxin reductase, NADPH-thioredoxin reductase, NADPH:oxidized thioredoxin oxidoreductase, thioredoxin reductase (NADPH)); CoA-glutathione reductase (EC 1.8.1.10, also known as CoA-glutathione reductase (NADPH), coenzyme A disulfide-glutathione reductase, coenzyme A glutathione disulfide reductase, NADPH-dependent coenzyme A-SS-glutathione reductase, NADPH:CoA-glutathione oxidoreductase); trypanothione-disulfide reductase (EC 1.8.1.12, also known as N(1),N(8)-bis(glutathionyl)spermidine reductase, NADPH:trypanothione oxidoreductase, trypanothione reductase); CoA-disulfide reductase (EC 1.8.1.14, also known as CoA-disulfide reductase (NAD(P)H), CoADR, coenzyme A disulfide reductase, NADH:CoA-disulfide oxidoreductase); mycothione reductase (EC 1.8.1.15, also known as mycothiol-disulfide reductase); protein-disulfide reductase (glutathione) (EC 1.8.4.2, also known as glutathione-insulin transhydrogenase, insulin reductase); Enzyme-thiol transhydrogenase (glutathione-disulfide) (EC 1.8.4.7, also known as [xanthine-dehydrogenase]: oxidized-glutathione S-oxidoreductase, enzyme-thiol transhydrogenase (oxidized-glutathione), glutathione-dependent thiol:disulfide oxidoreductase, thiol:disulfide oxidoreductase); CoB-CoM heterodisulfide reductase (EC 1.8.98.1, also known as heterodisulfide reductase, soluble heterodisulfide reductase); and/or protein disulfide-isomerase (EC 5.3.4.1, also known as S-S rearrangase). In the context of the invention, the term "reductase" is meant to comprise any of the above-mentioned disulfide-bridge altering enzymes.

In one embodiment of the invention, the enzyme in step (a*) is selected from the group consisting of one or more of cell wall-modifying enzyme, cell wall-degrading enzyme, protein-modifying enzyme, protein-degrading enzyme, fat-modifying enzyme, fat-degrading enzyme, disulfide bridge-modifying enzyme, disulfide bridge-oxidizing enzyme, disulfide-bridge-reducing enzyme, and any combination thereof. In a further embodiment, the enzyme is selected from the group consisting of one or more of cellulase, chitinase, amylase, protease, lipase, reductase and any combination thereof. In yet another embodiment, the enzyme is selected from one or more of any of the enzymes listed in this specification, including any combination thereof.

In the context of this invention, the term "spore" or "spores" relates to bacterial spore or spores unless stated otherwise. According to one embodiment of the invention, one or more of fungus, yeast, mould and/or fungal spore is/are successfully sterilized or disinfected.

In a further embodiment of the invention, the water-based fluid comprises - in addition to either (i) one or more reductant (s), (ii) one or more enzyme(s), or (iii) a combination of one or more reductant (s) and one or more enzyme(s) - one or more suitable additives, such as salt(s), coenzyme(s), trace element(s), stabilizing agent(s), buffering agent(s), polar, non-polar and zwitterionic detergent(s), surfactant, as well as antimicrobial agent(s) or antimicrobial composition(s). In a further embodiment, one or more enzyme(s) and/or one or more reductant(s) can be provided as a composition for example as powder or tablet, comprising 30% or more phosphate, 5-15% bleaching agents and less than 5% enzyme (percentages are weight percent in relation to the total weight of the composition). In another embodiment, one or more enzyme(s) and/or one or more reductant(s) can be provided as a composition, for example as powder or tablet, comprising 15-30% phosphate(s), less than 5% bleaches with oxygen, less than 5% nonionic tensides; less than 5% perfume (for example limonene), and than 5% enzyme (percentages are weight percent in relation to the total weight of the composition). In another embodiment, the water-based fluid containing at least one enzyme is provided by dissolving a composition, for example as powder or tablet, comprising 30% or more phosphate, 5-15% bleaching agents and less than 5% enzyme; or 15-30% phosphate(s), less than 5% bleaches with oxygen, less than 5% nonionic tensides; less than 5% perfume (for example limonene),and than 5% enzyme (percentages are weight percent in relation to the total weight of the composition). In yet another embodiment of the invention, the water-based fluid comprising one or more enzyme(s) and/or one or more reductant(s) is provided by diluting a concentrated stock solution. In yet a further embodiment, the water-based fluid comprising an enzyme is provided by dissolving a composition used for or suitable for household dishwashers.

A detergent according to the invention can be any polar- (i.e. ionic, anionic and/or cationic) non-polar-, or zwitterionic detergent or surfactant known in the art, including any combination of one or more polar, non-polar and/or zwitterionic detergent and/or surfactant. In one embodiment of the invention, a water based fluid comprises SDS (sodium dodecyl sulfate), in a concentration of about 0.5 to 2%, 0.1 to 0.5%, 0.05 to 0.1 %, 0.01 to 0.05%, or less than 0.01 % (weight/volume).

Examples of zwitterionic detergents according to the invention comprise, but are not limited to: 3-(N,N-dimethylmyristylammonio)propanesulfonate; 3-(N,N-dimethyloctylammonio)propanesulfonate inner salt; 3-(N,N-dimethyl-palmitylammonio)propanesulfonate; 3-(decyldimethylammonio)propanesulfonate inner salt; 3-[N,N-dimethyl(3-palmitoylaminopropyl)ammonio]-propanesulfonate; ASB-14; CHAPS (3-[(3-cholamidopropyl)dimethyl-ammonio]-1-propanesulfonate) CHAPSO; EMPIGEN® BB detergent; N-dodecyl-N,N-dimethyl-3-ammonio-1-propanesulfonate; and sodium 2,3-dimercaptopropanesulfonate monohydrate.

Examples of non-ionic detergents according to the invention comprise, but are not limited to: 2-cyclohexylethyl β-D-maltoside; 4-nonylphenoxypolyglycidyl ether; 6-cyclohexylhexyl β-D-maltoside; Bis(polyethylene glycol bis[imidazoyl carbonyl]); Brij® 30, 35, 56, 72, or 97; chenodeoxycholic acid diacetate methyl ester; Cremophor® EL; cyclohexylmethyl β-D-maltoside; decaethylene glycol mono-dodecyl ether; decyl β-D-glucopyranoside; decyl β-D-maltopyranoside; decyl-β-D-1-thioglucopyranoside; decyl-β-D-1-thiomaltopyranoside; diethylene glycol monodecyl ether; diethylene glycol monododecyl ether; diethylene glycol monohexadecyl ether; diethylene glycol monohexyl ether; diethylene glycol monooctyl ether; diethylene glycol monopentyl ether; diethylene glycol monotetradecyl ether; digitonin; digitoxigenin; dimethyldecylphosphine oxide; dodecyldimethylphosphine oxide; ethylene glycol monodecyl ether; ethylene glycol monododecyl ether; ethylene glycol monohexadecyl ether; ethylene glycol monohexyl ether; ethylene glycol monopentyl ether; glucopone; heptaethylene glycol monodecyl ether; heptaethylene glycol monododecyl ether; heptaethylene glycol monododecyl ether; heptaethylene glycol monohexadecyl ether; hexaethylene glycol monodecyl ether; hexaethylene glycol monododecyl ether; hexaethylene glycol monohexadecyl ether; hexaethylene glycol monooctadecyl ether; hexaethylene glycol monotetradecyl ether; hexyl β-D-glucopyranoside; IGEPAL® CA-630; methoxypolyethylene glycol 350; methyl 6-O-(N-heptylcarbamoyl)-a-D-glucopyranoside; N,N-dimethyloctadecylamine N-oxide; N-decanoyl-N-methylglucamine; N-octanoyl-β-D-glucosylamine; nonaethylene glycol monododecyl ether ; Nonidet™ P 40; nonyl β-D-glucopyranoside; nonyl-β-D-1-thiomaltoside; nonylphenyl-polyethyleneglycol acetate; octaethylene glycol monodecyl ether; octaethylene glycol monododecyl ether; octaethylene glycol monohexadecyl ether; octaethylene glycol monooctadecyl ether; octaethylene glycol monotetradecyl ether; octyl β-D-1-thioglucopyranoside; octyl β-D-glucopyranoside; pentaethylene glycol monodecyl ether; pentaethylene glycol monododecyl ether; pentaethylene glycol monohexadecyl ether; pentaethylene glycol monohexyl ether; pentaethylene glycol monooctyl ether; pentaethylene glycol monotetradecyl ether; poly(ethylene glycol) diglycidyl ether; poly(ethylene glycol); polyethylene glycol monomethyl ether; polyoxyethylene (20) sorbitan monolaurate; polyoxyethylene 10 tridecyl ether; polyoxyethylene 100 stearate; polyoxyethylene 20 oleyl ether; polyoxyethylene 40 stearate; polyoxyethylene 50 stearate; polyoxyethylene 8 stearate; polyoxyethylene 25 propylene glycol stearate; polysorbat; Pril®; SODOSIL®; saponin; Span®; steareth-100; sucrose monolaurate; TWEEN® 20, 21, 40, 60, 61, 65, 80, 81, or 85; Tergitol®; tetraethylene glycol monodecyl ether; tetraethylene glycol monododecyl ether; tetraethylene glycol monohexadecyl ether; tetraethylene glycol monooctadecyl ether; tetraethylene glycol monooctyl ether; tetraethylene glycol monotetradecyl ether; tetraethylenepentamine pentahydrochloride; tetramethylammonium hydroxide pentahydrate; triethylene glycol monodecyl ether; triethylene glycol monododecyl ether; triethylene glycol monoheptyl ether; triethylene glycol monooctyl ether; Triton® CF 10, N-57, N-60, X-100, X-102, X-114, X-15, X-165, X-207, X-305, X-405, X-45 , or X-705-70; tyloxapol; undecyl-β-D-maltoside; n-dodecyl β-D-maltoside; n-heptyl β-D-glucopyranoside; n-heptyl β-D-thioglucopyranoside; n-hexadecyl β-D-maltoside; and n-undecyl β-D-glucopyranoside.

Examples of anionic detergents according to the invention comprise, but are not limited to: 1-octanesulfonic acid sodium salt; chenodeoxycholic acid; cholic acid, e.g. from ox or sheep bile; dehydrocholic acid; deoxycholic acid; docusate sodium salt; glycocholic acid hydrate; glycodeoxycholic acid monohydrate; glycolithocholic acid ethyl ester; lithium dodecyl sulphate; lugol solution; N,N-dimethyldodecylamine N-oxide; N-lauroylsarcosine sodium salt; Niaproof™ 4; SODOSIL® RAM 05; SODOSIL® RM 002; SODOSIL® RM 003; SODOSIL® RM 01; SODOSIL® RM 02; sodium 1-butanesulfonate; sodium 1-decanesulfonate; sodium 1-dodecanesulfonate; sodium 1-heptanesulfonate; sodium 1-nonanesulfonate; sodium 1-propanesulfonate monohydrate; sodium chenodeoxycholate; sodium cholate hydrate; sodium choleate; sodium deoxycholate monohydrate; sodium deoxycholate; sodium dodecyl sulfate; sodium dodecylbenzenesulfonate; sodium glycochenodeoxycholate; sodium glycocholate hydrate; sodium glycodeoxycholate; sodium hexanesulfonate; sodium octyl sulfate; sodium pentanesulfonate; sodium taurochenodeoxycholate; sodium taurodeoxycholate hydrate; sodium taurohyodeoxycholate hydrate; sodium taurolithocholate; sodium tauroursodeoxycholate; taurocholic acid sodium salt hydrate; taurolithocholic acid 3-sulfate disodium salt; tetrabutylammonium perchlorate; Triton@ QS-15; Triton@ QS-44; Triton@ X-200; Triton@ XQS-20; Trizma® dodecyl sulfate; Turkey red oil sodium salt; and ursodeoxycholic acid.

Examples of anionic detergents according to the invention comprise, but are not limited to: alkyltrimethylammonium bromide; amprolium hydrochloride; benzalkonium chloride; benzethonium chloride; benzethonium hydroxide; benzyldimethylhexadecylammonium chloride; benzyldimethyltetradecyl-ammonium chloride; benzyldodecyldimethylammonium bromide; choline p-toluenesulfonate salt; denatonium benzoate; dimethyldioctadecylammonium bromide; dodecylethyldimethylammonium bromide; dodecyltrimethylammonium bromide; ethylhexadecyldimethylammonium bromide; irard's reagent T; hexadecylpyridinium bromide; hexadecylpyridinium chloride monohydrate; hexadecyltrimethylammonium bromide; hexadecyltrimethylammonium p-toluenesulfonate; Hyamine®; Luviquat™; methylbenzethonium chloride; myristyltrimethylammonium bromide; N,N',N'-Polyoxyethylene(10)-N-tallow-1,3-diaminopropane; oxyphenonium bromide; tetraheptylammonium bromide; tetrakis(decyl)ammonium bromide; and thonzonium bromide.

According to one embodiment of the invention, the water base fluid and/or the processed water comprises one or more additive(s) selected from the group consisting of one or more of salt, acid, base, buffering agent, coenzyme, trace element, stabilizing agent, polar detergent, non-polar detergent, zwitterionic detergent, antimicrobial composition, and any combination thereof.

In one embodiment of the present invention, a water based fluid is provided as cold steam and/or water vapour. This can be any water-based fluid of any of the steps (a) and (a*), as well as any rinsing step. One or more enzyme(s) and/or one or more reductant(s) and/or one or more additives can be dissolved or suspended in a water based fluid, which is applied in the form of cold steam vapor and/or as spray. Such a water-based fluid can comprise e.g. one or more reductant(s) and/or one or more enzyme(s) and/or one or more additives or other compounds can be applied in droplets, which can be comparable (e.g. in size) to droplets such as in steam or mist, or droplets produced by an atomizer known in the art. Without being bound to any theory, it is believed that the steam/mist/vapor/spray is likely to condensate and/or accumulate on surfaces, such as surfaces of an item to be sterilized according to the invention. Thereby a film of liquid, preferably a continuous film, is generated. Cold steam indicates also, that this "steam" is not hot, e.g. near or even above the boiling point of water, but at a significantly lower temperature, such as the temperatures provided according to the current invention.

The invention is not limited to a single, defined composition used for each step, and may be considered flexible and modular in terms of providing several different sterilization or disinfection protocols, according to e.g. the item or group of items to be sterilized, as well as depending on the microbial load, the time span available for sterilizing, tolerances towards various chemicals, temperatures, pressures, sizes and volumes which have to be treated.

Sterilization or disinfection according to the invention is a 4 log reduction of microorganisms, spores or microbial burden, more preferably a 5 log reduction and most preferably a 6 log reduction or even more. Sterilization is at least a 6 log reduction, disinfection at least a 4 log reduction, preferably 5 log reduction. Unless stated otherwise, sterilization or disinfection in the context of the present invention relates to surface sterilization or disinfection, respectively.

Another important feature of the present invention is the absence of higher temperatures during the sterilization or disinfection process. It can be desirable that the maximum surface temperature on the item to be sterilized or disinfected is below or not exceeding 100°C, 50°C, 37°C, 30°C, 25°C, 20°C, or ambient or room temperature which often is between 20°C and 25°C. The appropriate temperature or temperatures to be selected for the different steps of the invention depend on the efficiency of the different agents used in each step, combined with the temperature sensitivity of the objects or items to be sterilized, the desired level of disinfection (high, medium or low) and the total time available for the combined treatment.

According to one embodiment of the invention, each step (a), (a*) and/or (b) is performed using an individual temperature profile per step, said temperature profile being constant or varying or variable during said step. In another embodiment of the invention, all steps are performed at essentially the same temperature, such as at a temperature not exceeding 50°C, 37°C, 25°C, 20°C, or ambient temperature.

Different pressures (positive or negative) can be applied during the invention. In one embodiment, the absolute pressure applied is in the range of 1 to 300kPa, 10 to 200kPa, 50 to 150kPa, or 80 to 120kPa during any of the steps (a), (a*), (b) and/or any rinsing step(s). In one embodiment of the invention, a vacuum or reduced pressure is applied to facilitate providing an essentially water-free environment. In a further embodiment, the pressure applied corresponds to ambient pressure.

The method according to the invention allows for a rapid sterilization. According to one embodiment of the invention, the total time needed for sterilization is not exceeding 60 min, 30 min, 15 min, or 5 min. According to a further embodiment, sterilization or disinfection is achieved in less than 60 min; 30 min; 10 min; or 5 min. These time spans comprise all necessary steps for performing disinfection and/or sterilization from start to finish, thus including one or more step (a), (a*), (b) and any rinsing/washing step(s). According to another embodiment, step (a) is performed in not more than 20 min, 10 min, 8 min, 7 min, 6 min, 5 min, 4 min, 3 min, 2 min, or less than 1 min. According to a further embodiment, (a*) is performed in not more than 20 min; 10 min; 8 min, 7 min, 6 min, 5 min, 4 min, 3 min, 2 min, or 1 min, or between 20 and 30 min, 15 and 20 min, 10 and 15 min, 12 and 15 min, 10 and 12 min. According to yet another embodiment, step (b) is performed in not more than 20 min; 8 min, 7 min, 6 min, 5 min, 4 min, 3 min, 2 min, or 1 min. According to yet a further embodiment, a rinsing step with processed water or processed gas is performed in not more than 5 min, 4 min, 3 min, 2 min, 1 min, 45 sec, 30 sec, 15 sec, 10 sec, or 5 sec.

According to one embodiment of the invention, the total time needed for disinfection and/or sterilization is more than 60 min, such as between 1 hour and 2 hours, or more than 2 hours. In a further embodiment, the total time needed for disinfection and/or sterilization is in the range of 45-60 min, 30-45 min, 20-30min, 10-20 min, 5-10 min or 2-5 min.

According to still another embodiment, the total time of treatment with one or more water-based fluid(s), including any washing/rinsing step, i.e. the total time needed for step(s) (a), (a*) and any washing/rinsing step is less than 60 min; 40 min, 30 min; 20 min, 15 min, 10 min; or 5 min.

According to one embodiment of the invention, once treated according to the invention, the sterile items may be left in a suitable gas atmosphere, for storage until they are needed. Such a suitable gas can be selected from the group consisting of one or more of processed gas, ambient air, gas comprising one or more oxidant, ozone, gas comprising one or more reductant, nitrogen, inert as, helium, and any combination thereof.

Gases with oxidizing properties which may be used according to the invention may be selected and combined, without limitations from the group consisting of one or more oxygen, ozone, ethylene oxide, hydrogen peroxide, processed gas, ambient air, sterile ambient air, and any combination thereof.

In one embodiment of the invention, ozone is used as gas with oxidizing properties in a substantially water-free environment, such as by the use of an ozone generator known in the art. Ozone can be added to an appropriate gas, such as ambient air. In a further embodiment of the invention, ambient air is sterile, e.g by the use of UV light and/or filtration. In another embodiment of the invention, ozone concentrations are in the range of 1 to 10000 ppm, 5 to 100 ppm, 10 to 60 ppm, 40-60 ppm, 30-50 ppm, or 15-30 ppm. In a further embodiment of the invention, the ozone concentration is in the range of 1 to 1000 ppm, 10 to 600 ppm, 250 to 450 ppm, or around 350 ppm (i.e. 350 ppm +/-1, +/-2, +/-5 or +/-10 ppm).

The ozone concentration applied is dependent on the nature and degree of biological contamination, as well as the susceptibility to oxidative damage of the object to be sterilized.

Ozone in gas decays to O₂ with a half-life of approximately 3 days at 20 °C, but as fast as 1.5 seconds at 250 °C. This process may be accelerated by the use of known catalysts. Ozone may also be dissolved in water, where its half-life is considerably shorter, e.g. approximately 30 min at 15 °C or 8 min at or 35 °C at pH 7. Decomposition is faster in a basic environment, e.g. 3 min at pH 10.4 at 15 °C. Ozone may also be converted to oxygen by means of UV light.

This process may actually be utilized to sterilize the processed water used for sterilizing/disinfecting according to the invention, as well as to minimize ozone pollution during use of the method or apparatus according to the invention.

A second aspect of the invention concerns the use of the method as described above. This method may be used for treatment of one or more items selected from the group comprising laboratory items, medical items, dental items, veterinary items, military items, biological items and/or food processing related items. Furthermore, the method according to the invention may be used for satellites, space rockets and the like, where contamination of space, planets, asteroids, comets has to be avoided, for example in the context of investigating the question of presence or absence of extraterrestrial life forms.

An item or object or any part or parts thereof that is/are suitable for sterilization/disinfection according to the invention can be one or more that is/are sensitive to or impaired by one or more of temperature, pH, pressure, radiation and/or oxidation, or any combination thereof, e.g. when subjected to other forms of sterilization/disinfection. According to one embodiment of the invention, such an item or items, or part of said item can be selected from the group consisting of one or more laboratory item(s), medical item(s), dental item(s), military item(s), biological item(s), and food processing related item(s), and any combination thereof.

Such items or parts thereof could be selected from the group of medical instruments including instruments used for medical procedures in humans or animals including dental instruments. In one embodiment of the current invention, the item or part of an item to be sterilized/disinfected is an endoscope and/or ultrasound transducer.

According to one embodiment of the invention, an item or part of an item is a disposable item. In another embodiment, the item is a reusable item. Thus the invention is not limited to reusable items only, and disposable items may be processed as well by the use of the milder and gentler method of sterilization/disinfection according to the invention. In a further embodiment of the invention, disposable items are packaged/wrapped in a container or foil in an ozone containing atmosphere upon disinfection/sterilization, whereby they remain sterile/disinfected until use.

Another use of the method according to the invention is application during organ transplantation, such as surface treatment of tissues or organs of human or animal origin prior to implantation. A further use relates to sterilization or disinfection of one or more implants comprising pacemakers, joints, e.g. artificial hips, knees and the like, ligaments, bones, limbs or the like.

The method according to the invention is also suitable for decontamination of items, parts of items or surfaces, e.g. in the military context of fighting microbial warfare or after terrorist attack or suspicion of terrorist or military activities comprising microbial activities.

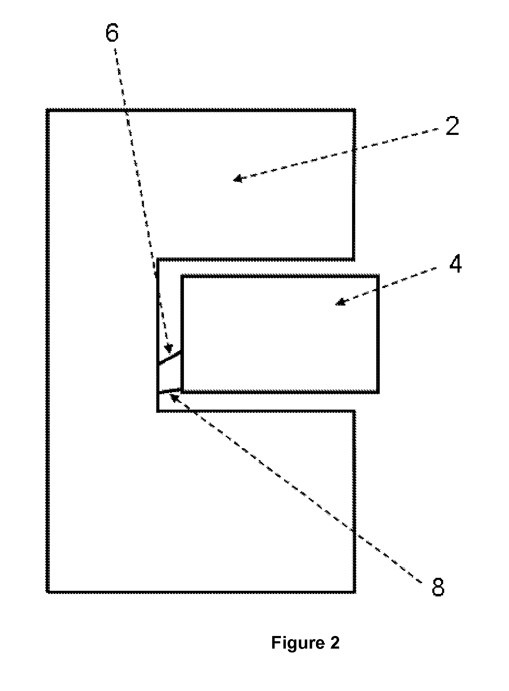

A third aspect of the invention relates to an apparatus comprising the necessary means for performing, using or applying the above mentioned method. In one embodiment a single, combined apparatus is used for performing steps of rendering a layer or fraction or subfraction of a layer or coat of the bacterial spore susceptible for the sporicidal/sterilizing action of the second treatment. In another embodiment, the invention is not limited to a single apparatus. Different devices may be used for the different steps according to the invention. For example, the rinsing and/or incubations step(s) can be performed in using one specialized apparatus, while the ozone treatment can be carried out in another device, and the time intervals in between the different steps may be selected accordingly. One embodiment of an apparatus according to the invention is shown in Figure 2.

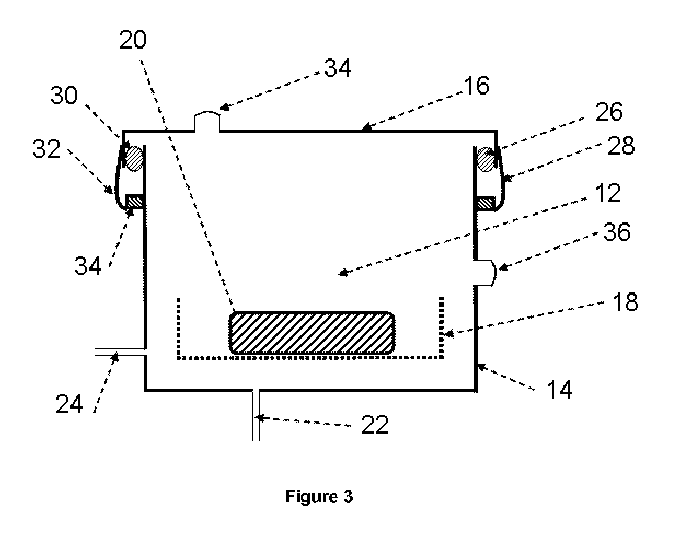

An apparatus according to the invention can also comprise one or more separate or individual reaction chamber(s) ("container(s)") for sterilizing one or more items (Fig. 3). Such a container may be removed from said apparatus, and more preferably, the content of such a container will remain sterile upon removal from said apparatus. If appropriate, the whole container, or just a part of it, for example the device (18) in Fig. 3 on which the item to be sterilized (20) rests, may be used in the transportation from one place to another, such as from one apparatus to another apparatus for performing one or more of the following treatments/steps in any order:
- a washing step with processed water, optionally comprising one or more additive(s);
- an incubation step with an aqueous fluid comprising a reductant and optionally one or more additive(s) and/or enzyme(s);
- an incubation step with an aqueous fluid comprising one or more enzyme(s) and/or additives;
- a rinsing step with processed water;
- a rinsing step with processed gas;
- an incubation step comprising treatment with an oxidizing gas.

In a further embodiment of the invention, the apparatus comprises pH-controlling means, pH-adjusting means and/or pH-maintaining means to control, adjust and/or maintain a defined pH in an aqueous fluid according to the invention. In one embodiment of the invention, said pH-controlling means, pH-adjusting means and/or pH-maintaining means can comprise a pH electrode and one or more titration means for dosing defined amounts of an acid, base or buffering agent, whereby a defined pH or a defined pH interval, e.g. a defined pH +/- 0.01, +/- 0.05, +/- 0.1, +/- 0.5 pH units can be maintained.

In an embodiment according to the invention, a pressure higher than ambient pressure is maintained in the container. Such a container can be equipped with a device or indicator of sterility, in an either manual, semi- or fully-automated fashion. Such a container can comprise a pressure indicator, indicating that the content of said container is sterilized.

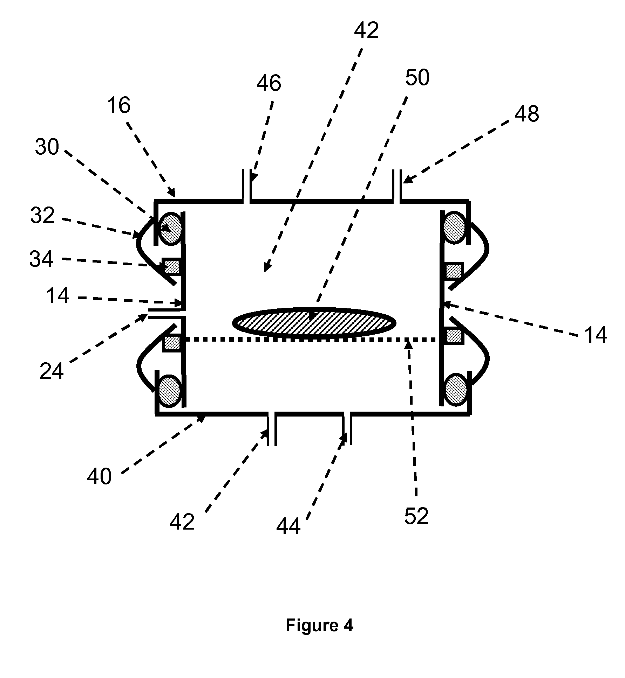

An alternative embodiment of a container according to the invention is illustrated schematically in Fig. 4. The container for sterilization and/or disinfection consists of a body (14) which is closed with two lids (16, 40). The lumen of the container is held air- and water tight by a seals, and clamping devices . The item or items to be treated (50) rest on a device (52), which is attached to the body (14). By removing both lids, one or several rinsing/washing/incubation steps can be performed, giving access to for washing/rinsing/treatment from top and bottom, if desired. Alternatively, the object/device to be treated, while situated on the device (52) surrounded by the body (14), may be placed in a rinsing/washing/incubation device. Once the lids are closed, the lumen (42) of the device can be filled with liquids and gasses via one or more inlets, outlets or in-and outlets (24, 43, 44, 46, 48), which can be situated either on the one or the other lid or both, or on the body (14).

### Brief description of the drawings

### Figure 1:

Schematic representation of a bacterial spore and the complex spore coat, not drawn to scale, modified from Setlow (2006). Note that the sizes of the layers of a spore may vary considerably between species. Ex: exosporium; SC: spore coat; OM: outer membrane; Cx: Cortex; GCW: germ cell wall; IM: inner membrane; Co: Core.

### Figure 2:

Schematic representation of an apparatus (2) according to the invention with a sterilizing chamber (4), one or more inlets (6) and one or more outlets (8).

### Figure 3:

Schematic representation of a sterilizing chamber according to the invention. A lumen (12) is created between the body of the sterilizing chamber (14) and a removable lid (16). Lid (16) and body (14) remain air-tightened by use of a seal (26, 30) and clamping device (28, 32, 34). The object to be sterilized (20) rests on a device (18), which may be detachable from the body (14).

Liquids and gases according to the invention are transported into and out of the lumen (12) by one or more inlets and outlets (22, 24). A pressure indicator located on either lid (34) or body of the sterilizing chamber (36) indicates if there is an overpressure present, indicating sterility of the item to be sterilized (20).

### Figure 4:

Schematic representation of an alternative disinfection/sterilizing chamber according to the invention. A lumen (42) is created between two lids (16 and 40) and the body of the sterilizing chamber (14). As in Fig. 3, the container is air- and water tightened by seals (30) and one or more clamping devices (32, 34). For simplicity, not all of the depicted seals and clamping devices are numbered. The item or object to be sterilized or disinfected (50) rests on a device (52), which is attached to the body (14). By removing both lids, one or several rinsing/washing/incubation steps can be performed, giving access to washing/rinsing/treatment from top and bottom if desired. The lumen (42) of the device can be filled with liquids and/or gasses via one or more inlets, outlets or in-and outlets (24, 43, 44, 46, 48), which can be situated either on the one or the other lid or both, or on the body (14). In the present example, only of one of the potentially several in/outlet(s) (24) are shown.

### Figure 5:

Tryptone Soya Agar plates showing colony growth on filters with spores after treatment with DMG buffer (top), DMG buffer + 10 mM DTT (middle), and DMG buffer + 10 mM DTT + 1% (weight/volume) SDS (bottom), followed by 5 or 10 min treatment with 350 ppm ozone. Experiments were performed in duplicate. The control filter half without ozone treatment is shown to the left.

### EXAMPLES

### Experiment 1

A material compatibility test was performed to investigate ozone's corrosivity with respect to materials commonly used for medical equipments, such as ultrasound transducers and flexible endoscopes. Following materials were selected as representatives of different groups of materials: ABS polyurethane TPX, silicone, neoprene rubber and PVC. Materials were tested in a chamber with 40 to 50 ppm (parts per million) ozone at 25°C to 40°C for 80 hours subjected to cycles of one hour in ozone and one hour in air. There were no visible damages or changes to the surface of the material compared to untreated control samples.

### Experiment 2

In a model system, the sterilization and/or disinfection effect was investigated of a treatment comprising a liquid comprising a reductant and a detergent, followed by a treatment with ozone gas.

*Bacillus atrophaeus* spores are highly resistant to heat and chemicals and their use is officially recognized for sterilization procedure certification. Similarly suture loops and porcelain penicylinders inoculated with *Bacillus subtilis* spores are used to determine the efficiency of sterilization protocols.

### Materials and Methods:

Bacterial spores: Stainless steel discs with *Bacillus atrophaeus* spores (log 6, cat# 1-6100ST, Raven Biological Laboratories).
Polyester suture loops and porcelain penicylinders are inoculated with
*Bacillus subtilis* strain ATCC19659 (Presque Isle Cultures).
Buffer and chemicals:
DMG = 3,3-dimethylglutaric acid (D-4379, Sigma-Aldrich)
DTT = DL-dithiotreitol (D-0632, Sigma Aldrich)
SDS = sodium dodecyl sulfate (UN1325, BDH) 10% SDS = 10% (weight/volume) SDS in destilled or deionized water
DMG buffer = 50 mM DMG, pH 7.0 in destilled or deionized water water

All solutions were sterilized by autoclaving or sterile filtration using common laboratory techniques.

### Growth medium:

Standard growth media were used, such as Nutrient Agar (i.e. Peptone, Meat extract and Agar), or TSA (i.e. Tryptone Soya Agar)

### Ozone generator:

The ozone generator provided by the applicant provided ∼350 ppm O₃, and a flow rate of ∼25 l/min, using setting No. 1.

Filtration:

Commercially available sterile single filters (pore size 0,45 µm) and an analytical test filter funnel from Nalgene was used (F-2161, Sigma-Aldrich). For suction, a Rietschle Thomas DTF 6 vacuum pump was used, with a flow rate of ~6 m³/h, providing a vacuum of ~150 mbar abs.

### Experimental Procedures:

*Filtering test*: In filtering test experiments the spore solutions were prepared in a volume of 1 ml and processed. Thereto, after an incubation period of 5 min in the appropriate buffer at 40°C using a rotary shaker, an aliquot of 250 µl of the suspension was transferred onto a sterile single use filters. The filter was washed two times with 10 ml of sterile water. The filter was retrieved under sterile conditions, and the filter was cut into halves using a sterile scissor. Only one half of the filter was treated with ozone, while the other half was kept as control, i.e. without ozone treatment. Finally, both halves were placed onto a TSA plate and incubated at 30°C. Initially Nutrient Agar plates were used, but later on a switch was made to TSA plates, in order to utilize the more pronounced reddish colour of *Bacillus atrophaeus* colonies which was facilitating analysis and documentation.

In filtering test experiments a sample was diluted and plated prior to filtering (dilution series prepared in sterile water) in order to determine the number of live bacterial spores in the filtered solution.

*Sterility test*: In sterility test experiments sutures and penicylinders are individually placed in glass tubes containing 5 ml Nutrient Broth and incubated for 1-2 days at 30°C with shaking. Visual inspection of the tubes is used to monitor growth/no growth. The length of the lag phase, i.e. the time period before growth can be observed, is an indicator of the number of viable cells. The longer the lag phase, the lower the number of viable cells, indicating killing, sterilizing or disinfection of the inoculated suture and/or penicylinder.

### Results:

The following 3 test solutions/buffers were tested using 2 ml eppendorf tubes.
1 ml DMG buffer
990 µl DMG buffer + 10 µl 1 M DTT
890 µl DMG buffer + 10 µl 1 M DTT + 100 µl 10% SDS
   Spore disks were added to the tubes using either procedure A (rotary shaker at 40°C, 250 µl sample filtered) and incubated. The applicant had established that the use of a strong vacuum pump, providing a vacuum of ∼150 mbar abs, improved the recovery percentage of spores significantly, most likely, because of a more efficient removal of water.

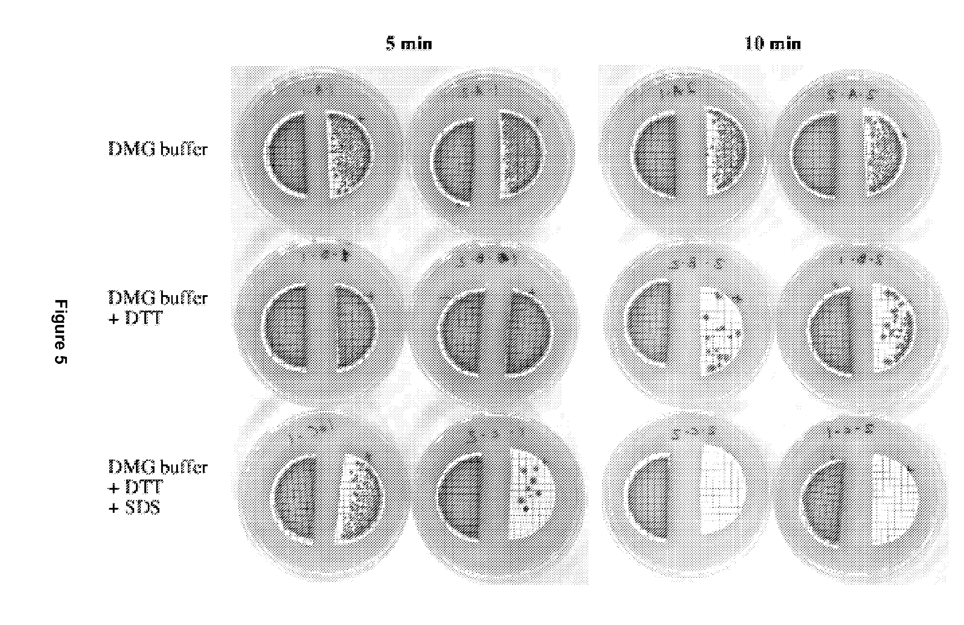

Filter halves were incubated for 5 or 10 min with ozone (∼350 ppm O₃ in ambient filtered and sterilized ambient air with ambient humidity, estimated to be in the range of between 30-60 (%)), and placed on growth medium as described above. Figure 5 shows the results of such an experiment (2 repetitions), and the results are further summarized in Table I. The control filter halve (without ozone treatment) is seen to the left of the ozone treated halve.

Longer ozone treatment times were found to increase the killing effect. For both procedures, killing of spores was increased after 10 min compared to 5 min. In all experiments, the control filter halves which were not treated with ozone revealed significant bacterial growth, proving the importance of a consecutive ozone treatment step.
The sterilizing/disinfecting/killing effect of ozone was found to be improved for solutions containing DTT compared to the control (DMG buffer alone).

Further addition of SDS compared to the control (DMG buffer alone) revealed the highest reduction in cfu, indicating a further increase of in the sterilizing/disinfecting/killing effect of ozone.

*Reference experiment (recovery*/*total number of spores)*: Prior to filtration a spore suspension sample was diluted 100 times and plated (100 µl) onto agar medium plates (Nutrient Agar or TSA) to determine the number of viable spores. Plates were incubated at 30°C for 1-2 days before counting the number of colonies.

Approximately 1200 colonies per plate were observed (average value for all plates using four repetitions). Because 100 µl were plated, this corresponds to 12.000 colonies per ml of the 100 fold diluted spore suspension, thus a total of 1.2×10⁶ spores/ml prior to filtration. As the spore discs used in the experiments are of the log 6 type, the above obtained colony counts indicate a complete recovery of the bacterial spores from the discs.

***Conclusion:*** Treatment of spores in a liquid media comprising a reductant (DTT), followed by treatment with sterile air comprising ∼350 ppm ozone for 5 or 10 min was able to reduce viability of *Bacillus atrophaeus* by 3-5 logs. The most pronounced killing effect was obtained using a combination of DTT + SDS in the test solution mixture.

**Table I. Comparison of colony counts (cfu) on filter halves (2 repetitions) of treatment with buffer, buffer + reductant, and buffer + reductant + detergent (see also Figure 5).**

| | 5 min O₃ | 10 min O₃ |
|---|---|---|
| DMG* | ∼800 | ∼100 |
| DMG + DTT** | ∼1200 | ∼25 |
| DMG +DTT + SDS*** | ∼100 | 0 |

| | | |
|---|---|---|
| *1 ml DMG buffer **990 µl DMG buffer + 100 µl 1 M DTT ***890 µl DMG buffer + 100 µl 1 M DTT + 100 µl 10% SDS | | |

## Claims

1. A method for sterilization or disinfection, comprising the steps of contacting one or more item or part of said item with:
(a) a water-based fluid comprising at least one reductant;
(b) a substantially water-free environment with a gas having oxidative properties;
wherein said step (a) precedes step (b).

2. A method according to claim 1, comprising a step (a*) of contacting one or more item or part of said item with a water-based fluid comprising at least one enzyme, wherein said step (a*) precedes step (b).

3. A method according to claim 2, wherein step (a*) precedes step (a).

4. A method according to claim 2, wherein step (a) precedes step (a*).

5. A method according to any of the preceding claims, wherein said step (a) and/or (a*) comprise(s) an ultrasonic treatment.

6. A method according to any of the preceding claims, wherein the water based fluid is provided as cold steam and/or water vapour.

7. A method according to any one of the preceding claims, wherein one or more rinsing step(s) is included before step (a), step (a*), and/or step (b).

8. A method according to claim 7, wherein said rinsing step comprises contacting said one or more item or part of said item with processed water and/or processed gas.

9. A method according to any one of the preceding claims, wherein said reductant is capable of reducing a disulfide bridge between two cysteins.

10. A method according to claim 9, wherein the reductant is one or more of dithiothreitol, a derivate of dithiothreitol, β-mercaptoethanol, and/or a derivate of β-mercaptoethanol.

11. A method according to any of the preceding claims, wherein the water base fluid and/or the processed water comprises one or more additive(s) selected from the group consisting of one or more of salt, acid, base, buffering agent, coenzyme, trace element, stabilizing agent, polar detergent, non-polar detergent, zwitterionic detergent, and antimicrobial composition.

12. A method according to any one of the preceding claims, wherein said gas having oxidative properties is ozone.

13. A method according to claim 12, wherein the ozone concentration is in the range of 1 to 1000 ppm, 10 to 600 ppm, 250 to 450 ppm, or around 350 ppm.

14. A method according to any one of the claims 2 to 13, wherein said enzyme in step (a*) is selected from the group consisting of one or more of cell wall-modifying enzyme, cell wall-degrading enzyme, protein-modifying enzyme, protein-degrading enzyme, fat-modifying enzyme, fat-degrading enzyme, disulfide bridge-modifying enzyme, disulfide bridge-oxidizing enzyme, disulfide-bridge-reducing enzyme.

15. A method according to claim 11, wherein said enzyme is selected from the group consisting of one or more of cellulase, chitinase, amylase, protease, lipase, and reductase.

16. A method according to any one of the preceding claims, wherein the maximum temperature is not exceeding 50°C, 37°C, 25°C, 20°C, or ambient temperature.

17. A method according claim 16, wherein each step (a), (a*) and/or (b) is performed using an individual temperature profile per step, said temperature profile being constant or varying or variable during said step.

18. A method according to any one of the preceding claims, wherein said step (a) is performed at a pH in the range of pH 6.0 to 14.0; 7.0 to 10; or 7.0 to 8.0.

19. A method according to any one of the preceding claims, wherein said step (a*) is performed at a pH in the range of pH 4 to 10; 6 to 8; or 6.8 to 7.2.

20. A method according to any one of the preceding claims, wherein the pressure applied is in the range of 1 to 300 kPa; 10 to 200 kPa; 50 to 150 kPa; or 80 to 120kPa during any of the steps (a), (a*), (b) and/or any rinsing step(s).

21. A method according to any of the preceding claims, wherein step (a) is performed in less than 20 min; 10 min; 5 min; or 1 min.

22. A method according to any of the preceding claims, wherein step (a*) is performed in less than 20 min; 10 min; 5 min; or 1 min.

23. A method according to any of the preceding claims, wherein step (b) is performed in less than 20 min; 10 min; 5 min; or 1 min.

24. A method according to any of the preceding claims, wherein sterilization or disinfection is achieved in less than 60 min; 30 min; 10 min; or 5 min.

25. A method according to any of the preceding claims, wherein said one or more item or part of said one or more item is selected from one or more of laboratory item, medical item, dental item, sanitary item, military item, biological item, and/or food processing-related item.

26. A method according to claim 25, wherein said item or part of said item is sensitive to, or impaired by one or more of temperature, pH, positive or negative pressure, radiation and/or oxidation.

27. A method according to claim 25 or 26, wherein said one or more item or part of said one or more item is a disposable item.

28. A method according to claim 25 or 26, wherein said one or more item or part of said one or more item is an endoscope.

29. An apparatus adapted to perform the method according to any one of the preceding claims.

30. An apparatus according to claim 29, comprising a reaction chamber adapted to sterilizing said one or more item or part of said one or more item.
